# FASCICULE DE BREVET EUROPEEN

(11) **EP 2 095 810 B1**
(45) Date de publication et mention de la délivrance du brevet: **01.06.2016**
(21) Numéro de dépôt: 09153329.9
(22) Date de dépôt: 20.02.2009
(51) Int. Cl.: A61K 8/891, A61Q 5/12

(54) **Composition cosmétique comprenant un copolymère siliconé particulier, un solvant volatil et une résine de silicone particulière**
Kosmetische Zusammensetzung enthaltend ein spezifisches Silikonpolymer, ein flüchtiges Lösungsmittel und ein spezifisches Silikonharz
Cosmetic composition comprising a particular silicone copolymer, a volatile solvent and a particular silicone resin

(30) Priorité: 26.02.2008 FR 0851215
(43) Date de publication de la demande: 02.09.2009
(73) Titulaire: L'Oréal, 75008 Paris (FR)
(72) Inventeur: Brun, Gaëlle, 75015 PARIS (FR); Bonnamy, Arnaud, 78000 VERSAILLES (FR)
(74) Mandataire: Prevel, Estelle Nicole

(56) Documents cités:
- WO-A-2005/075567
- WO-A-2007/051505
- US-A1- 2006 110 346
- US-B2- 6 991 782

## Description

La présente invention a pour objet une composition de traitement des fibres kératiniques, et en particulier des fibres kératiniques humaines telles que les cheveux, ainsi qu'un procédé de traitement des fibres kératiniques mettant en oeuvre cette composition.

Les cheveux sont généralement abîmés et fragilisés par l'action des agents atmosphériques extérieurs tels que la lumière et les intempéries, et par des traitements mécaniques ou chimiques tels que le brossage, le peignage, les décolorations, les permanentes et / ou les teintures. Il en résulte que les cheveux sont souvent difficiles à discipliner, en particulier ils sont difficiles à démêler ou à coiffer, et les chevelures, même abondantes, conservent difficilement une coiffure de bon aspect en raison du fait que les cheveux manquent de vigueur, de volume et de nervosité.

Cette dégradation des cheveux est par ailleurs accrue par la répétition de traitements de coloration permanente des cheveux, qui consiste à appliquer sur les cheveux un ou plusieurs précurseurs de colorant et un agent oxydant.

Ainsi, pour remédier à cela, il est maintenant usuel d'utiliser des produits de coiffage qui permettent de conditionner les cheveux en leur apportant notamment du corps, de la masse ou du volume.

Ces produits de coiffage sont généralement des compositions cosmétiques capillaires comprenant un ou plusieurs polymères qui présentent une forte affinité pour les cheveux et qui ont le plus souvent pour fonction de former un film à leur surface en vue de modifier leurs propriétés superficielles, notamment pour les conditionner ou pour leur apporter des propriétés optiques particulières.

Un inconvénient lié à l'utilisation de ces compositions capillaires réside dans le fait que les effets cosmétiques conférés par de telles compositions ont tendance à disparaître, notamment dès le premier shampooing. Ceci est d'autant plus vrai lorsqu'un des effets conférés est un effet coloriel apporté par des pigments.

Afin de remédier à cet inconvénient, il est envisageable d'accroître la rémanence du dépôt de polymères en effectuant directement une polymérisation radicalaire de certains monomères au niveau des cheveux. Toutefois, les traitements ainsi obtenus entraînent une dégradation de la fibre et les cheveux ainsi traités sont généralement difficilement démélables.

Il est par ailleurs connu d'effectuer des gainages des cheveux à partir d'une composition comprenant un monomère électrophile de type cyanoacrylate, notamment dans la demande de brevet FR 2 833 489. Une telle composition permet d'obtenir des cheveux parfaitement gainés et non gras. Cependant, le gainage obtenu nécessite des conditions opératoires particulières dues à la réactivité du monomère électrophile. Par ailleurs, le gainage obtenu avec ces monomères électrophiles devient collant avec les corps gras tels que le sébum.
Il existe aussi des copolymères de silicone particuliers qui comprennent un segment résine de silicone et un segment silicone fluide, plus connus sous le nom de BioPSA. Ces copolymères sont notamment décrits dans les demandes de brevet WO 03/026596, WO 2004/073626, WO 2007/051505 et WO 2007/051506 pour diverses applications cosmétiques telles que l'application sur les cheveux, les ongles, la peau. Le toucher obtenu avec ces copolymères est généralement collant. On connaît également du document US 2006/0110346 une composition comprenant une silicone adhésive sensible à la pression, une huile volatile et une résine triméthyl siloxysilicate (résine de type MQ), qui ne comprend pas d'unité trifonctionnelle (CH₃)SiO_{3/2}.

Ainsi, le but de la présente invention est de mettre au point un procédé de traitement des fibres kératiniques, et en particulier des fibres kératiniques humaines telles que les cheveux, facile de mise en oeuvre qui permet d'obtenir des gainages rémanents aux shampoings et aux diverses agressions que peuvent subir les cheveux, notamment les brushings et la transpiration tout en tolérant mieux les corps gras tels que le sébum et en ne développant pas de caractère collant. Notamment, le but de la présente invention est d'obtenir des gainages colorés, non collants, faciles à mettre en oeuvre, résistants aux agents extérieurs et respectant l'intégrité des fibres kératiniques.

Ce but est atteint avec la présente invention qui a pour objet une composition de traitement des fibres kératiniques comprenant un ou plusieurs copolymères à base de résine de silicone et de silicone fluide, un ou plusieurs solvants volatils, et une ou plusieurs résines de silicone ayant au moins une unité trifonctionnelle de formule (CH₃)SiO_{3/2} (unité T).

L'invention a aussi pour objet un procédé de traitement des fibres kératiniques pour l'obtention d'un effet rémanent aux shampoings mettant en oeuvre cette composition.

Un autre objet de l'invention est l'utilisation de cette composition pour l'obtention d'un gainage coloré ou non sur les fibres kératiniques.

Ces gainages permettent d'obtenir du volume, de la masse et du corps aux cheveux de façon rémanente aux shampooings tout en préservant les qualités physiques de la fibre kératiniques, avec de plus un effet coloriel rémanent lorsque la composition contient des pigments. Un tel gainage est en particulier résistant aux agressions extérieures que peuvent subir les cheveux telles que le brushing et la transpiration. Il permet de plus d'obtenir des colorations permanentes sans utilisation d'agents oxydants susceptibles de dégrader les cheveux.

Le gainage ainsi formé se présente sous forme d'un dépôt homogène, lisse et possède une excellente adhésion sur cheveux. Par ailleurs, on a constaté de façon surprenante que les cheveux restaient parfaitement individualisés, pouvaient être coiffés sans problème et que les propriétés de coiffant apportées à la fibre étaient rémanentes aux shampooings. De plus, le toucher obtenu est peu chargé et non collant.

Dans ce qui suit, sauf indication contraire, les bornes des intervalles indiqués sont comprises dans l'invention.

### Copolymère à base de résine de silicone et de silicone fluide

Le copolymère siliconé défini selon l'invention est dérivé de la réaction entre une résine de silicone et une silicone fluide.

De tels copolymères sont décrits par exemple dans « Silicone Pressure Sensitive Adhesive », Sobieski and Tangney, Handbook of Pressure Sensitive Adhesive Technology (D. Satas Ed.), Von Nostrand Reinhold, New York.

Dans le copolymère, la résine de silicone est présente à un taux compris entre 45 et 75 % (par rapport à la masse totale de silicone) et la silicone fluide est présente à un taux compris entre 25 et 55 %, avec la somme des pourcentages de résine de silicone et de silicone fluide qui est égal à 100. De préférence, la résine de silicone est présente à un taux compris entre 55 et 65 % (par rapport à la masse totale de silicone) et la silicone fluide est présente à un taux compris entre 35 et 45 %, avec la somme des pourcentages de résine de silicone et de silicone fluide qui est égal à 100.

De préférence, la résine de silicone selon l'invention est le produit de condensation de groupements SiO₂ et de groupements R₃(SiO)_{1/2} (triorganosilyle) pour lequel chaque groupement R est indépendamment sélectionné parmi les radicaux méthyle, éthyle, propyle ou vinyle et pour lequel le rapport entre les fonctions SiO₂ et les fonctions R₃(SiO)_{1/2} de la résine de silicone va de 0,6 à 0,9. Des groupements triorganosilyle utilisables pour former la résine de silicone peuvent être des unités triméthylsilyle, triéthylsilyle, méthylméthylproprylsilyle, diméthylvinylsilyle et des mélanges de celles-ci. Le groupement triméthylsilyle est le préféré dans le cadre de l'invention.

De préférence, la silicone fluide selon l'invention est une diorganopolysiloxane aux fonctions OH terminales ayant une viscosité comprise entre 100 et 100 000 est à 25 °C pour laquelle les substituants de la diorganopolysiloxane sont indépendamment choisis parmi les radicaux méthyle, éthyle, propyle ou vinyle. Les diorganopolysiloxanes sont de préférence des polymères linéaires. Des exemples de diorganopolysiloxane peuvent être, de manière non limitative, une polydiméthylsiloxane, une éthylméthylpolysiloxane, un copolymère de diméthylsiloxane et de méthylvinylsiloxane, et des mélanges de tels polymères ou copolymères ayant des extrémités OH. La diorganopolysiloxane préférée est une polydiméthylsiloxane.

Des exemples de synthèse d'un tel copolymère sont par exemple décrits dans le brevet US 5 162 410 ou dans le brevet CA 711756.

Les copolymères selon la présente invention peuvent donc être préparés en chauffant le mélange suivant :
- de 45 % à 75 % en masse de résine de silicone étant le produit de condensation des unités SiO₂ et R₃(SiO)_{1/2} pour lequel chaque groupement R est indépendamment sélectionné parmi les radicaux méthyle, éthyle, propyle ou vinyle et pour lequel le rapport entre les fonctions SiO₂ et les fonctions R₃(SiO)_{1/2} de la résine de silicone va de 0,6 à 0,9 ;
- de 25 % à 55 % en masse de diorganopolysiloxane fluide aux fonctions OH terminales ayant une viscosité comprise entre 100 et 100 000 est à 25 °C pour laquelle les substituants de la diorganopolysiloxane sont indépendamment choisis parmi les radicaux méthyle, éthyle, propyle ou vinyle ;
- de 0,001 % à 5 % d'un catalyseur adapté, qui est de préférence un composé aminé aliphatique organique choisi de préférence parmi les amines primaires, les amines secondaires, les amines tertiaires, les sels d'acide carboxylique des amines citées ci-dessus et les sels d'ammonium quaternaires.

Le mélange est chauffé à une température comprise entre 80 °C et 160 °C jusqu'à ce que le caractère adhésif du copolymère siliconé résultant soit obtenu.

Les copolymères préférés selon l'invention sont commercialisés par Dow Corning sous la référence BIO-PSA^{®}, ces BIO-PSA^{®} pouvant être sous deux formes, standard ou amine compatible, et étant fournis dans différents solvants avec plusieurs ratio résine de silicone / silicone fluide. On peut notamment citer les grades 7-4400, 7-4500, 7-4600. Le BIO-PSA^{®} particulièrement préféré selon l'invention est le grade 7-4400.

Selon un mode de réalisation particulier de l'invention, la quantité de copolymère est supérieure à 1 % en poids du poids total de la composition.

Le copolymère peut notamment être présent dans la composition selon l'invention en une teneur supérieure à 1 % et jusqu'à 40 % en poids par rapport au poids total de la composition, de préférence allant de 1,5 % à 20 % en poids, et préférentiellement allant de 1,5 % à 15 % en poids.

### Solvant volatil

Selon l'invention, la composition appliquée sur les cheveux contient un ou plusieurs solvants volatils.

Dans le cadre de l'invention, on entend par solvant volatil un composé liquide à la température ambiante (20 °C) et à la pression atmosphérique (760 mmHg) présentant une pression de vapeur à 20 °C supérieure à 0,1 mmHg et de préférence comprise entre 0,1 et 300 mmHg, encore plus préférentiellement entre 0,5 et 200 mmHg.

Ce solvant volatil peut être un solvant organique non siliconé, un solvant organique siliconé ou leurs mélanges.

A titre de solvant organique non siliconé volatil, on peut citer:
- les alcanols volatils en C₁-C₄ tels que l'éthanol, l'isopropanol ;
- les alcanes volatils en C₅-C₇ tels que le n-pentane, l'hexane, le cyclopentane, le 2,3-diméthylbutane, le 2,2-diméthylbutane, le 2-méthylpentane, le 3-méthylpentane ;
- les esters d'acides en C₁-C₂₀ liquides et d'alcools en C₁-C₈ volatils tels que l'acétate de méthyle, l'acétate de n-butyle, l'acétate d'éthyle, l'acétate de propyle, l'acétate d'isopentyle, le 3-éthoxypropionate d'éthyle ;
- les cétones liquides à température ambiante et volatiles telles que la méthyléthylcétone, la méthylisobutylcétone, la diisobutylcétone, l'isophorone, la cyclohexanone, l'acétone ;
- les polyols volatils tels que le propylène glycol ;
- les éthers volatils tels que le diméthoxyméthane, le diéthoxyéthane, le diéthyléther ;
- les éthers de glycol volatils comme le 2-butoxyéthanol, le butyle diglycol, le monométhyléther de diéthylène glycol, le n-butyléther de propylène glycol, l'acétate de monométhyl éther de propylène glycol ;
- les huiles hydrocarbonées volatiles telles que les huiles volatiles hydrocarbonées ayant de 8 à 16 atomes de carbone et leurs mélanges, et notamment les alcanes ramifiés en C₈-C₁₆ comme les iso-alcanes (appelées aussi isoparaffines) en C₈-C₁₆, l'isododécane, l'isodécane, et par exemple les huiles vendues sous les noms commerciaux d'Isopars ou de Permetyls, et leurs mélanges. On peut aussi citer les néopentanoate d'isohexyle ou d'isodecyle ;
- les perfluoroalcanes volatils en C₄-C₁₀ tels que le dodécafluoropentane, le tétradécafluorohexane, le décafluoropentane ;
- les perfluorocycloalkyles volatils tels que le perfluorométhylcyclopentane, le 1,3-perfluorodiméthylcyclohexane et le perfluorodecaline, vendus respectivement sous les dénominations de "Flutec PC1®", "Flutec PC3®" et "Flutec PC6®" par la Société F2 Chemicals, ainsi que le perfluorodiméthylcyclobutane et la perfluoromorpholine ;
- les composés fluoroalkyles ou hétérofluoroalkyles volatils répondant à la formule suivante :

   CH₃-(CH₂)ₙ-[Z]ₜ-X-CF₃
dans laquelle t est 0 ou 1 ; n est 0, 1, 2 ou 3 ; X est un radical perfluoroalkyle divalent, linéaire ou ramifié, ayant de 2 à 5 atomes de carbone, et Z représente O, S, ou NR, R étant un atome d'hydrogène, un radical -(CH₂)ₙ-CH₃ ou un radical -(CF₂)ₘ-CF₃, m étant 2, 3, 4 ou 5.

Parmi les composés fluoroalkyles ou hétérofluoroalkyles volatils, on peut notamment citer le méthoxynonafluorobutane vendu sous la dénomination de "MSX 4518®", "HFE-7100®" par la Société 3M et l'éthoxynonafluorobutane vendu sous la dénomination de "HFE-7200®" par la Société 3M.

De préférence, le solvant est choisi de telle manière que son point d'ébullition soit inférieur à 200 °C.

Selon un mode de réalisation particulier, le solvant organique non siliconé est choisi parmi l'éthanol, l'isopropanol, l'acétone, les alcanes liquides à 25 °C et à pression atmosphérique (760 mmHg) tels que l'isododécane.

A titre de solvant siliconé volatil, on peut citer les composés siliconés à faible viscosité choisi parmi les silicones linéaires ou cycliques ayant de 2 à 7 atomes de silicium, ces silicones comportant éventuellement des groupes alkyle ou alkoxy ayant de 1 à 10 atomes de carbone, par exemple l'octaméthylcyclotétrasiloxane, la décaméthylcyclopentasiloxane, la dodécaméthylcyclohexasiloxane, l'heptaméthylhexyltri-siloxane, l'heptaméthyléthyltrisiloxane, l'heptaméthyloctyltrisiloxane, l'octaméthyltrisiloxane, la décaméthyltétrasiloxane, et leurs mélanges. Selon un mode de réalisation particulier, le composé siliconé est choisi parmi la cyclopentadiméthylsiloxane et la dodécaméthylcyclohexasiloxane.

Selon un mode de réalisation particulier, le solvant siliconé volatil présente une viscosité inférieure à 50 centistokes.

De préférence, la silicone volatile est choisie parmi la décaméthylcyclopentasiloxane, la dodécaméthylcyclohexasiloxane, l'octaméthyltrisiloxane et la décaméthyltétrasiloxane.

A titre d'exemple, on peut citer la décaméthylcyclopentasiloxane commercialisée sous le nom DC-245 par la société Dow Corning, la dodécaméthylcyclohexasiloxane commercialisée sous le nom DC-246 par la société Dow Corning, l'octaméthyltrisiloxane commercialisée sous le nom DC-200 Fluid 1 cst par la société Dow Corning et la décaméthyltétrasiloxane commercialisée sous le nom DC-200 Fluid 1,5 est par la société Dow Corning.

Cette silicone volatile présente généralement une viscosité faible, par exemple une viscosité inférieure à 10 cst à 25 °C.

De préférence, la silicone volatile est cyclique et est la décaméthylcyclopentasiloxane commercialisée sous le nom DC-245 par la société Dow Corning ou la dodécaméthylcyclohexasiloxane commercialisée sous le nom DC-246 par la société Dow Corning.

Le solvant volatil peut être présent dans la composition utile dans le procédé de l'invention en une teneur allant de 0,1 % à 95 % en poids par rapport au poids total de la composition, de préférence allant de 1 % à 70 % en poids, et préférentiellement allant de 5 % à 90 % en poids.

### Résine de silicone comprenant au moins une unité T

Par le terme « résine », on entend dans le cadre de l'invention une structure tridimensionnelle réticulée ou non. A titre d'exemple de résine de polysiloxane, on peut citer les silsesquioxanes et les siloxysilicates.

La nomenclature des résines de silicone est connue sous le nom de "MDTQ", la résine étant décrite en fonction des différentes unités monomèriques siloxane qu'elle comprend, chacune des lettres "MDTQ" caractérisant un type d'unité.

La lettre M représente l'unité monofonctionelle de formule (CH₃)₃SiO_{1/2}, l'atome de silicium étant relié à un seul atome d'oxygène dans le polymère comprenant cette unité.

La lettre D signifie une unité difonctionnelle (CH₃)₂SiO_{2/2} dans laquelle l'atome de silicium est relié à deux atomes d'oxygène.

La lettre T représente une unité trifonctionnelle de formule (CH₃)SiO_{3/2}.

Dans les motifs M, D, T définis précédemment, au moins un des groupes méthyle peut être substitués par un groupe R différent du groupe méthyle tel qu'un radical hydrocarboné (notamment alkyle) ayant de 2 à 10 atomes de carbone ou un groupe phényle ou bien encore un groupe hydroxyle.

Enfin, la lettre Q signifie une unité tétrafonctionnelle SiO_{4/2} dans laquelle l'atome de silicium est lié à quatre atomes d'hydrogène eux-mêmes liés au reste du polymère.

Diverses résines de propriétés différentes peuvent être obtenues à partir de ces différentes unités, les propriétés de ces polymères variant en fonction du type de monomères (ou unités), du type et du nombre de radicaux substitués, de la longueur de la chaîne polymérique, du degré de ramification et de la taille des chaines pendantes.

La résine de silicone selon l'invention contient au moins une unité T. Elle peut donc être par exemple une résine T, MT, MTQ et MDTQ.

De préférence, la résine de silicone contient au moins 50 % d'unités T, et encore plus préférentiellement au moins 80 % d'unités T.

Les résines T préférées selon l'invention peuvent contenir des unités M, D et Q de manière à ce que au moins 80 mol % voire au moins 90 mol % par rapport au total des silicones soient des unités T. Les résines T peuvent également contenir des groupements hydroxy et / ou alcoxy. Généralement les résines T ont un poids total de fonctions hydroxy compris entre 2 et 10 % et un poids total de fonctions alcoxy pouvant aller jusqu'à 20 %, de préférence le poids total de fonctions hydroxy est compris entre 4 et 8 % et le poids total de fonctions alcoxy peut aller jusqu'à 10 %.

De préférence la résine de silicone est choisie parmi les silsesquioxanes qui sont représentées par la formule suivante : ((CH₃)SiO_{3/2})ₓ, où x peut aller jusqu'à plusieurs milliers et le radical CH₃ peut être remplacé par un radical R, comme décrit précédemment dans la définition des unités T. De préférence le nombre x d'unités T du silsesquioxane est inférieur ou égal à 500, il est plus préférentiellement compris entre 50 et 500. Le poids moléculaire de la résine de silicone selon l'invention est donc de préférence compris entre 500 et 50 000 g/mol, plus préférentiellement compris entre 500 et 20 000 g/mol et encore plus préférentiellement compris entre 500 et 10 000 g/mol.

La résine de silicone selon l'invention est de préférence filmogène. En effet tous les silsesquioxanes ne sont pas filmogènes, par exemple les polyméthylsilsesquioxanes hautement polymérisées comme les TospearlTM de Toshiba ou la KMP590 de Shin-Etsu sont insolubles et ne sont pas filmogènes. Le poids moléculaire des ces polyméthylsilsesquioxanes est difficile à déterminer mais il y a généralement plus de 1000 unités T.

A titre d'exemples de ces résines silicones contenant au moins une unité T, on peut citer :
- les polysilesquioxanes de formule (CH₃SiO_{3/2})ₓ (unités T) dans laquelle x est supérieur à 100 et dont au moins un des radicaux méthyle peut être substitué par un groupement R tel que défini plus haut ;
- les polyméthylsilsesquioxanes qui sont des polysilsesquioxanes dans lesquels aucun des radicaux méthyle n'est substitué par un autre groupement. De tels polymethylsilsesquioxanes sont décrits dans le document US 5 246 694 dont le contenu est incorporé par référence ;
- les polypropylsilsesquioxanes, pour lesquelles les radicaux méthyle sont remplacés par des radicaux propyle. Ces composés ainsi que leur synthèse sont notamment décrit dans la demande de brevet WO 2005/075567 ;
- les polyphénylsilsesquioxanes, pour lesquelles les radicaux méthyle sont remplacés par des radicaux phényle. Ces composés ainsi que leur synthèse sont notamment décrit dans la demande de brevet US 2004/0180011.

A titre d'exemples de résines polyméthylsilsesquioxanes commercialement disponibles, on peut citer celles qui sont commercialisées :
- par la société Wacker sous la référence Resin MK tels que la Belsil PMS MK : polymère comprenant des unités répétitives CH₃SiO_{3/2} (unités T), pouvant aussi comprendre jusqu'à 1 % en poids d'unités (CH₃)₂SiO_{2/2} (unités D) et présentant un poids moléculaire moyen d'environ 10 000 g/mol. On pense que le polymère se trouve dans une configuration « cage » et « échelle » comme cela est représenté dans les figures ci-dessous. Le poids moléculaire moyen des unités en configuration « cage » a été calculé à 536 g/mol. La majorité du polymère se trouve en configuration « échelle » avec des groupes éthoxy aux extrémités. Ces groupes éthoxy représentent 4,5 % en masse du polymère. Comme ces extrémités peuvent réagir avec l'eau, un taux faible et variable de groupes SiOH peut également être présent. 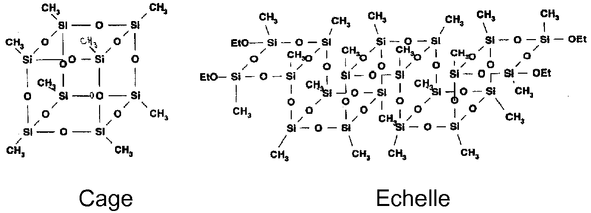
- par la société SHIN-ETSU sous les références KR-220L qui sont composées d'unités T de formule CH₃SiO_{3/2} et ont des groupes terminaux Si-OH (silanol), sous la référence KR-242A qui comprennent 98 % d'unités T et 2 % d'unités diméthyle D et ont des groupes terminaux Si-OH ou encore sous la référence KR-251 comprenant 88 % d'unités T et 12 % d'unités diméthyle D et ont des groupes terminaux Si-OH.

A titre d'exemples de résines polypropylsilsesquioxanes commercialement disponibles, on peut citer celles qui sont commercialisées :
- par la société DOW CORNING sous la référence Dow Corning 670 Fluid qui est une polypropylsilsesquioxane diluée dans la D5.

A titre d'exemples de résines polyphénylsilsesquioxanes commercialement disponibles, on peut citer celles qui sont commercialisées :
- par la société DOW CORNING sous la référence Dow Corning 217 Flake Resin qui est une polyphénylsilsesquioxane aux extrémités silanol ;
- par la société WACKER sous la référence Belsil SPR 45 VP.

Dans un mode de réalisation de l'invention, les résines de silicones utiles dans la composition de l'invention sont solubles ou dispersables dans la composition de l'invention. De préférence, les résines de silicone selon l'invention sont solubles dans les silicones volatiles et les solvants organiques. Dans un mode de réalisation, la résine de silicone est solide à 25 °C.

La ou les résines de silicone comprenant au moins une unité T peuvent être présentes dans la composition à une concentration allant de 0,1 à 20 %, de préférence de 0,2 à 15 % et encore plus préférentiellement de 0,5 à 10 %.

Selon un mode de réalisation particulier de l'invention, le rapport pondéral entre les résines de silicone comprenant au moins une unité T et les solvants volatils est compris entre 1/200 et 1/10.

### PDMS linéaire non volatile de viscosité supérieure à 5 cst

Selon un mode de réalisation particulier, la composition comprend une ou plusieurs PDMS linéaires non volatiles de viscosité supérieure à 5 cst afin d'améliorer l'homogénéité du gainage.

La polydiméthylsiloxane (PDMS) linéaire non volatile de viscosité supérieure à 5 cst est notamment une gomme de silicone ou une huile de silicone de pression de vapeur inférieure à 0,1 mm Hg à 25 °C.

La PDMS linéaire non volatile de viscosité supérieure à 5 cst peut être choisie parmi les polydiméthylsiloxanes ; les alkyldiméthicones ; les polyphénylméthylsiloxanes tels que les phényldiméthicones, les phényltriméthicones, et les vinylméthylméthicones ; ainsi que les silicones modifiées par des groupements aliphatiques et / ou aromatiques, éventuellement fluorés, ou par des groupements fonctionnels tels que des groupements hydroxyle, thiol et / ou amine.

De préférence, la viscosité des PDMS linéaires non volatiles utiles dans la présente invention est supérieure à 5 cst à 25 °C. Selon un mode de réalisation particulier, cette viscosité est comprise entre 5 cst et 5 000 000 cst, de préférence entre 100 cst et 4 000 000 et encore plus préférentiellement de 5000 et 4 000 000 cst.

Le poids moléculaire est généralement compris entre 500 et 800 000 g/mol, de préférence de 5000 à 700 000 g/mol et encore plus préférentiellement de 50 000 à 600 000 g/mol.

Cette PDMS linéaire peut notamment être choisie parmi les silicones de formule (I): 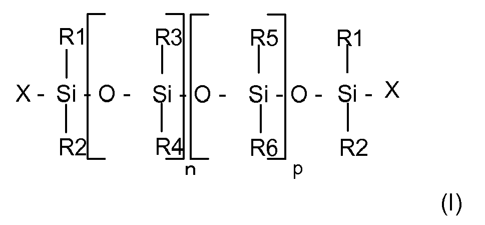 dans laquelle :
R₁, R₂, R₅ et R₆ sont, ensemble ou séparément, un radical alkyle ayant 1 à 6 atomes de carbone ;
R₃ et R₄ sont, ensemble ou séparément, un radical alkyle ayant de 1 à 6 atomes de carbone, un radical vinyle ou un radical aryle ;
X est un radical alkyle ayant de 1 à 6 atomes de carbone, un radical hydroxyle ou un radical vinyle, un radical amine ;
n et p étant des entiers choisis de manière à avoir un composé de viscosité supérieure à 5 cst, de préférence la somme n + p est supérieure à 10.

A titre d'exemple, on peut citer les polydiméthylsiloxanes suivantes :
- les substituants R₁ à R₆ et X représentent un groupement méthyle, comme celle vendue sous la dénomination Baysilicone TP 3898 par la société Général Electric, et celle vendue sous la dénomination AK 500000 par la société Waker ;
- les substituants R₁ à R₆ et X représentent un groupement méthyle, p et n sont tels que le poids moléculaire est de 120 000 g/mol, comme celle vendue sous la dénomination Dow Corning 200 Fluid 60000 CS par la société Dow Corning ;
- les substituants R₁ à R₆ et X représentent un groupement méthyle, p et n sont tels que le poids moléculaire est de 250 000 g/mol, comme celle vendue sous la dénomination Mirasil DM 500.000 par la société Rhodia et celle vendue sous la dénomination Dow Corning 200 Fluid 500 000 cst par la société Dow Corning ;
- les substituants R₁ à R₆ représentent un groupement méthyle, le groupement X représente un groupement hydroxy, n et p sont tels que le poids moléculaire du polymère soit de 600 000 g/mol, comme celle vendue sous la dénomination SGM 36 par la société Dow Corning ;
- les diméthicones du type (polydiméthylsiloxane)(méthylvinylsiloxane) telle que la SE63 commercialisée par GE BAYER Silicones, les copolymères poly(diméthylsiloxane)(diphényl)(méthylvinylsiloxane), et leurs mélanges.

De préférence, les PDMS linéaires non volatiles ne sont oxyalkylénés.

Lorsqu'elles sont présentes, la quantité en PDMS linéaires non volatiles est généralement comprise entre 0,1 à 20 %, de préférence entre 0,5 à 10 %.

### Solvant organique non volatil

La composition conforme à l'invention peut également comprendre un ou plusieurs solvants organiques non volatils.

A titre de solvant organique non volatil, on peut citer :
- les alcools aromatiques non volatils tels que l'alcool benzylique, le phénoxyéthanol ;
- les esters d'acides en C₁-C₂₀ liquides et d'alcools en C₁-C₈ non volatils tels que le myristate d'isopropyle ;
- l'éthylène carbonate, le propylène carbonate, le butylène carbonate ;
- les polyols non volatils tels que le glycérol, l'éthylène glycol, le dipropylène glycol, le butylène glycol ;
- les éthers de glycol non volatils comme le monoéthyléther de diéthylène glycol, le mono n-butyl éther de dipropylène glycol ;
- les huiles hydrocarbonées non volatiles telles que l'isohexadécane ;
- les alcools gras liquides non volatils en C₁₀-C₃₀ tels que l'alcool oléique, les esters d'alcools gras en C₁₀-C₃₀ liquides tels que les benzoates d'alcool gras en C₁₀-C₃₀ et leurs mélanges ; l'huile de polybutène, l'isononanoate d'isononyle, le malate d'isostéaryle, le tétra-isostéarate de pentaérythrityle, le trimélate de tridécyle ;
- les solvants perfluorés non volatils tels que le perfluoroperhydrophénanthrène, vendu sous la dénomination de "Flutec PC11®" par la Société F2 Chemicals.

Le ou les solvants organiques non volatils peuvent être présents dans la composition selon l'invention en une teneur allant de 0,1 % à 90 % en poids par rapport au poids total de la composition, de préférence allant de 1 % à 80 % en poids, et préférentiellement allant de 5 % à 70 % en poids.

### Pigments

Selon une variante, la composition est une composition de coloration des fibres kératiniques qui comprend des pigments. Une telle composition permet d'obtenir des gainages rémanents, colorants et ceci sans dégradation des fibres kératiniques.

Par pigment, on entend tous les pigments apportant de la couleur aux matières kératiniques. Leur solubilité dans l'eau à 25 °C et à pression atmosphérique (760 mmHg) est inférieure à 0,05 %, et de préférence inférieure à 0,01 %.

Les pigments qui peuvent être utilisés sont notamment choisis parmi les pigments organiques et / ou minéraux connus de la technique, notamment ceux qui sont décrits dans l'encyclopédie de technologie chimique de Kirk-Othmer et dans l'encyclopédie de chimie industrielle de Ullmann.

Ces pigments peuvent se présenter sous forme de poudre ou de pâte pigmentaire. Ils peuvent être enrobés ou non enrobés.

Les pigments peuvent par exemple être choisis parmi les pigments minéraux, les pigments organiques, les laques, les pigments à effets spéciaux tels que les nacres ou les paillettes, et leurs mélanges.

Le pigment peut être un pigment minéral. Par pigment minéral, on entend tout pigment qui répond à la définition de l'encyclopédie Ullmann dans le chapitre pigment inorganique. On peut citer, parmi les pigments minéraux utiles dans la présente invention, les oxydes de fer ou de chrome, le violet de manganèse, le bleu outremer, l'hydrate de chrome et le bleu ferrique, le dioxyde de titane.

Le pigment peut-être un pigment organique. Par pigment organique, on entend tout pigment qui répond à la définition de l'encyclopédie Ullmann dans le chapitre pigment organique. Le pigment organique peut notamment être choisi parmi les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

En particulier, les pigments organiques blancs ou colorés peuvent être choisis parmi le carmin, le noir de carbone, le noir d'aniline, le jaune azo, la quinacridone, le bleu de phtalocyanine, le rouge sorgho, les pigments bleus codifiés dans le Color Index sous les références CI 42090, 69800, 69825, 73000, 74100, 74160, les pigments jaunes codifiés dans le Color Index sous les références CI 11680, 11710, 15985, 19140, 20040, 21100, 21108, 47000, 47005, les pigments verts codifiés dans le Color Index sous les références CI 61565, 61570, 74260, les pigments oranges codifiés dans le Color Index sous les réfénces CI 11725, 15510, 45370, 71105, les pigments rouges codifiés dans le Color Index sous les références CI 12085, 12120, 12370, 12420, 12490, 14700, 15525, 15580, 15620, 15630, 15800, 15850, 15865, 15880, 17200, 26100, 45380, 45410, 58000, 73360, 73915, 75470, les pigments obtenus par polymérisation oxydante de dérivés indoliques, phénoliques tels qu'ils sont décrits dans le brevet FR 2 679 771.

A titre d'exemple on peut aussi citer les pâtes pigmentaires de pigment organique telles que les produits vendus par la société HOECHST sous le nom :
- JAUNE COSMENYL IOG : Pigment YELLOW 3 (CI 11710) ;
- JAUNE COSMENYL G : Pigment YELLOW 1 (CI 11680) ;
- ORANGE COSMENYL GR : Pigment ORANGE 43 (CI 71105) ;
- ROUGE COSMENYL R : Pigment RED 4 (CI 12085) ;
- CARMIN COSMENYL FB : Pigment RED 5 (CI 12490) ;
- VIOLET COSMENYL RL : Pigment VIOLET 23 (CI 51319) ;
- BLEU COSMENYL A2R : Pigment BLUE 15.1 (CI 74160) ;
- VERT COSMENYL GG : Pigment GREEN 7 (CI 74260) ;
- NOIR COSMENYL R : Pigment BLACK 7 (CI 77266).

Les pigments conformes à l'invention peuvent aussi être sous forme de pigments composites tels qu'ils sont décrits dans le brevet EP 1 184 426. Ces pigments composites peuvent être composés notamment de particules comportant un noyau inorganique, au moins un liant assurant la fixation des pigments organiques sur le noyau, et au moins un pigment organique recouvrant au moins partiellement le noyau.

Le pigment organique peut aussi être une laque. Par laque, on entend les colorants adsorbés sur des particules insolubles, l'ensemble ainsi obtenu restant insoluble lors de l'utilisation.

Les substrats inorganiques sur lesquels sont adsorbés les colorants sont par exemple l'alumine, la silice, le borosilicate de calcium et de sodium ou le borosilicate de calcium et d'aluminium, et l'aluminium.

Parmi les colorants, on peut citer le carmin de cochenille. On peut également citer les colorants connus sous les dénominations suivantes : D & C Red 21 (CI 45 380), D & C Orange 5 (CI 45 370), D & C Red 27 (CI 45 410), D & C Orange 10 (CI 45 425), D & C Red 3 (CI 45 430), D & C Red 4 (CI 15 510), D & C Red 33 (CI 17 200), D & C Yellow 5 (CI 19 140), D & C Yellow 6 (CI 15 985), D & C Green (CI 61 570), D & C Yellow 1 O (CI 77 002), D & C Green 3 (CI 42 053), D & C Blue 1 (CI 42 090).

A titre d'exemples de laques, on peut citer le produit connu sous la dénomination suivante : D & C Red 7 (CI 15 850:1).

Le pigment peut aussi être un pigment à effets spéciaux. Par pigments à effets spéciaux, on entend les pigments qui créent d'une manière générale une apparence colorée (caractérisée par une certaine nuance, une certaine vivacité et une certaine clarté) non uniforme et changeante en fonction des conditions d'observation (lumière, température, angles d'observation...). Ils s'opposent par-là même aux pigments colorés qui procurent une teinte uniforme opaque, semi-transparente ou transparente classique.

Il existe plusieurs types de pigments à effets spéciaux, ceux à faible indice de réfraction tels que les pigments fluorescents, photochromes ou thermochromes, et ceux à plus fort indice de réfraction tels que les nacres ou les paillettes.

A titre d'exemples de pigments à effets spéciaux, on peut citer les pigments nacrés tels que le mica recouvert de titane, ou d'oxychlorure de bismuth, les pigments nacrés colorés tels que le mica recouvert de titane et d'oxydes de fer, le mica recouvert d'oxyde de fer, le mica recouvert de titane et notamment de bleu ferrique ou d'oxyde de chrome, le mica recouvert de titane et d'un pigment organique tel que défini précédemment ainsi que les pigments nacrés à base d'oxychlorure de bismuth. A titre de pigments nacrés, on peut citer les nacres Cellini commercialisée par Engelhard (Mica-TiO₂-laque), Prestige commercialisée par Eckart (Mica-TiO₂), Prestige Bronze commercialisée par Eckart (Mica-Fe₂O₃), Colorona commercialisée par Merck (Mica-TiO₂-Fe₂O₃).

En plus des nacres sur un support mica, on peut envisager les pigments multicouches basés sur des substrats synthétiques comme l'alumine, la silice, le borosilicate de calcium et de sodium ou le borosilicate de calcium et d'aluminium, et l'aluminium.

On peut également citer les pigments à effet interférentiel non fixés sur un substrat comme les cristaux liquides (Helicones HC de Wacker), les paillettes holographiques interférentielles (Geometric Pigments ou Spectra f/x de Spectratek). Les pigments à effets spéciaux comprennent aussi les pigments fluorescents, que ce soit les substances fluorescentes à la lumière du jour ou qui produisent une fluorescence ultraviolette, les pigments phosphorescents, les pigments photochromiques, les pigments thermochromiques et les quantum dots, commercialisés par exemple par la société Quantum Dots Corporation.

Les quantum dots sont des nanoparticules semi conductrices luminescentes capables d'émettre, sous excitation lumineuse, un rayonnement présentant une longueur d'onde comprise entre 400 nm et 700 nm. Ces nanoparticules sont connues de la littérature. En particulier, elles peuvent être synthétisées selon les procédés décrits par exemple dans le US 6 225 198 ou US 5 990 479, dans les publications qui y sont citées, ainsi que dans les publications suivantes : Dabboussi B.O. et al "(CdSe)ZnS core-shell quantum dots : synthesis and characterisation of a size series of highly luminescent nanocristallites" Journal of phisical chemistry B, vol 101, 1997, pp 9463-9475 et Peng, Xiaogang et al, "Epitaxial Growth of highly Luminescent CdSe/CdS core/shell nanocrystals with photostability and electronic accessibility" Journal of the American Chemical Society, vol 119, N°30, pp 7019-7029.

La variété des pigments qui peuvent être utilisés dans la présente invention permet d'obtenir une riche palette de couleurs, ainsi que des effets optiques particuliers tels que des effets métalliques, interférentiels.

La taille du pigment utilisé dans la composition cosmétique selon la présente invention est généralement comprise entre 10 nm et 200 µm, de préférence entre 20 nm et 80 µm, et plus préférentiellement entre 30 nm et 50 µm.

Les pigments peuvent être dispersés dans le produit grâce à un agent dispersant.

L'agent dispersant sert à protéger les particules dispersées contre leur agglomération ou floculation. Cet agent dispersant peut être un tensioactif, un oligomère, un polymère ou un mélange de plusieurs d'entre eux, portant une ou des fonctionnalités ayant une affinité forte pour la surface des particules à disperser. En particulier, ils peuvent s'accrocher physiquement ou chimiquement à la surface des pigments. Ces dispersants présentent, en outre, au moins un groupe fonctionnel compatible ou soluble dans le milieu continu. En particulier, on utilise les esters de l'acide hydroxy-12 stéarique en particulier et d'acide gras en C₈ à C₂₀ et de polyol comme le glycérol, la diglycérine, tel que le stéarate d'acide poly(12-hydroxystéarique) de poids moléculaire d'environ 750 g/mole tel que celui vendu sous le nom de Solsperse 21 000 par la société Avecia, le polygycéryl-2 dipolyhydroxystéarate (nom CTFA) vendu sous la référence Dehymyls PGPH par la société Henkel ou encore l'acide polyhydroxystéarique tel que celui vendu sous la référence Arlacel P100 par la société Uniqema et leurs mélanges.

Comme autre dispersant utilisable dans les compositions de l'invention, on peut citer les dérivés ammonium quaternaire d'acides gras polycondensés comme le Solsperse 17 000 vendu par la société Avecia, les mélanges de polydiméthylsiloxane / oxypropylène tels que ceux vendus par la société Dow Corning sous les références DC2-5185, DC2-5225 C.

Les pigments utilisés dans la composition cosmétique selon l'invention peuvent être traités en surface par un agent organique.

Ainsi les pigments préalablement traités en surface utiles dans le cadre de l'invention sont des pigments qui ont subi totalement ou partiellement un traitement de surface de nature chimique, électronique, électro-chimique, mécano-chimique ou mécanique, avec un agent organique tel que ceux qui sont décrits notamment dans Cosmetics and Toiletries, Février 1990, Vol. 105, p. 53-64 avant d'être dispersés dans la composition conforme à l'invention. Ces agents organiques peuvent être par exemple choisis parmi les acides aminés ; les cires, par exemple la cire de carnauba et la cire d'abeille ; les acides gras, les alcools gras et leurs dérivés, tels que l'acide stéarique, l'acide hydroxystéarique, l'alcool stéarylique, l'alcool hydroxystéarylique, l'acide laurique et leurs dérivés ; les tensio-actifs anioniques ; les lécithines ; les sels de sodium, potassium, magnésium, fer, titane, zinc ou aluminium d'acides gras, par exemple le stéarate ou laurate d'aluminium ; les alcoxydes métalliques ; les polysaccharides, par exemple le chitosane, la cellulose et ses dérivés ; le polyéthylène ; les polymères (méth)acryliques, par exemple les polyméthylmethacrylates ; les polymères et copolymères contenant des motifs acrylates ; les protéines ; les alcanoamines ; les composés siliconés, par exemple les silicones, les polydiméthylsiloxanes, les alcoxysilanes, les alkylsilanes, les siloxy-silicates ; les composés organiques fluorés, par exemple les perfluoroalkyle éthers ; les composés fluoro-siliconés.

Les pigments traités en surface utiles dans la composition cosmétique selon l'invention peuvent aussi avoir été traités par un mélange de ces composés et / ou avoir subi plusieurs traitements de surface.

Les pigments traités en surface utiles dans le cadre de la présente invention peuvent être préparés selon des techniques de traitement de surface bien connues de l'homme de l'art ou trouvés tels quels dans le commerce.

De préférence, les pigments traités en surface sont recouverts par une couche organique.

L'agent organique avec lequel sont traités les pigments peut être déposé sur les pigments par évaporation de solvant, réaction chimique entre les molécules de l'agent de surface ou création d'une liaison covalente entre l'agent de surface et les pigments. Le traitement en surface peut ainsi être réalisé par exemple par réaction chimique d'un agent de surface avec la surface des pigments et création d'une liaison covalente entre l'agent de surface et les pigments ou les charges. Cette méthode est notamment décrite dans le brevet US 4 578 266.

De préférence, on utilisera un agent organique lié aux pigments de manière covalente.

L'agent pour le traitement de surface peut représenter de 0,1 à 50 % en poids du poids total des pigments traités en surface, de préférence de 0,5 à 30 % en poids, et encore plus préférentiellement de 1 à 10 % en poids.

De préférence, les traitements en surface des pigments sont choisis parmi les traitements suivants :
- un traitement PEG-Silicone comme le traitement de surface AQ commercialisé par LCW;
- un traitement Chitosane comme le traitement de surface CTS commercialisé par LCW;
- un traitement Triéthoxycaprylylsilane comme le traitement de surface AS commercialisé par LCW ;
- un traitement Méthicone comme le traitement de surface SI commercialisé par LCW ;
- un traitement Diméthicone comme le traitement de surface Covasil 3.05 commercialisé par LCW ;
- un traitement Diméthicone / Triméthylsiloxysilicate comme le traitement de surface Covasil 4.05 commercialisé par LCW ;
- un traitement Lauroyl Lysine comme le traitement de surface LL commercialisé par LCW;
- un traitement Lauroyl Lysine Diméthicone comme le traitement de surface LL / SI commercialisé par LCW ;
- un traitement Myristate de Magnésium comme le traitement de surface MM commercialisé par LCW ;
- un traitement Dimyristate d'Aluminium comme le traitement de surface MI commercialisé par Miyoshi ;
- un traitement Perfluoropolyméthylisopropyl éther comme le traitement de surface FHC commercialisé par LCW ;
- un traitement Isostéaryl Sébacate comme le traitement de surface HS commercialisé par Miyoshi ;
- un traitement Disodium Stéaroyl Glutamate comme le traitement de surface NAI commercialisé par Miyoshi ;
- un traitement Diméthicone / Disodium Stéaroyl Glutamate comme le traitement de surface SA / NAI commercialisé par Miyoshi ;
- un traitement Phosphate de Perfluoroalkyle comme le traitement de surface PF commercialisé par Daito ;
- un traitement Copolymère acrylate / Diméthicone et Phosphate de Perfluoalkyle comme le traitement de surface FSA commercialisé par Daito ;
- un traitement Polyméthylhydrogène siloxane / Phosphate de Perfluoroalkyle comme le traitement de surface FS01 commercialisé par Daito ;
- un traitement Lauryl Lysine / Aluminium Tristéarate comme le traitement de surface LL-StAI commercialisé par Daito ;
- un traitement Octyltriéthylsilane comme le traitement de surface OTS commercialisé par Daito ;
- un traitement Octyltriéthylsilane / Phosphate de Perfluoroalkyle comme le traitement de surface FOTS commercialisé par Daito ;
- un traitement Copolymère Acrylate / Diméthicone comme le traitement de surface ASC commercialisé par Daito ;
- un traitement Isopropyl Titanium Triisostéarate comme le traitement de surface ITT commercialisé par Daito ;
- un traitement Cellulose Microcrystalline et Carboxyméthyl Cellulose comme le traitement de surface AC commercialisé par Daito ;
- un traitement Cellulose comme le traitement de surface C2 commercialisé par Daito ;
- un traitement copolymère Acrylate comme le traitement de surface APD commercialisé par Daito ;
- un traitement Phosphate de Perfluoroalkyle / Isopropyl Titanium Triisostéarate comme le traitement de surface PF + ITT commercialisé par Daito.

La composition conforme à la présente invention peut de plus comprendre un ou plusieurs pigments non traités en surface.

Lorsqu'ils sont présents, la quantité de pigments est généralement comprise entre 0,1 à 40 % en poids, de préférence 0,5 à 20 % en poids du poids total de la composition.

### Composés additionnels

La composition de l'invention peut contenir d'autres espèces colorées ou colorantes telle que des colorants directs hydrophiles ou hydrophobes ou des précurseurs de colorants.

La composition de l'invention peut contenir des composés siliconés particuliers autres que ceux décrits précédemment, notamment les diméthiconols tels que la DC1501 Fluid.

La composition de l'invention peut ainsi contenir un polymère siliconé greffé. Dans le cadre de l'invention, on entend par polymère siliconé greffé, un polymère comprenant une portion polysiloxane et une portion constituée d'une chaîne organique non-siliconée, l'une des deux portions constituant la chaîne principale du polymère, l'autre étant greffée sur ladite chaîne principale.

Les polymères siliconés greffés utilisés dans la composition cosmétique selon l'invention sont choisis préférentiellement dans le groupe constitué par les polymères à squelette organique non siliconé greffé par des monomères contenant un polysiloxane, les polymères à squelette polysiloxanique greffé par des monomères organiques non siliconés et leurs mélanges.

La composition de l'invention peut aussi comprendre un polymère non siliconé qui permet d'améliorer soit les propriétés intrinsèques de la composition, soit le gainage obtenu lors de l'application sur le cheveu, soit les deux.

Un tel polymère peut être choisi parmi les polymères suivants :
- les polymères solubles dans un milieu liquide organique, en particulier les polymères liposolubles ;
- les polymères dispersibles dans un milieu solvant organique, en particulier les polymères sous la forme de dispersions non aqueuses de particules de polymères de taille primaire inférieure à 1 µm, de préférence des dispersions dans les huiles siliconées ou hydrocarbonées ;
- les polymères sous forme de dispersions aqueuses de particules de polymère de taille primaire inférieure à 1 µm, souvent appelées « latex » ; dans ce cas, la composition comprend une phase aqueuse ;
- les polymères hydrosolubles ; dans ce cas, la composition comprend une phase aqueuse ou bien le polymère est appliqué en pré ou post-traitement de la composition selon l'invention.

Le polymère utilisable dans la composition peut être anionique, cationique, non-ionique ou amphotère.

La composition peut aussi contenir des charges qui sont généralement des composés substantiellement non colorés solides à température ambiante et pression atmosphérique (760 mmHg), et insolubles dans la composition, même lorsque ces ingrédients sont portés à une température supérieure de la température ambiante.

Les charges peuvent être minérales ou organiques. Les charges peuvent être des particules de toute forme, notamment plaquettaires, sphériques ou oblongues, quelle que soit leur forme cristallographique (par exemple feuillet, cubique, hexagonale, orthorhombique). De plus ces particules peuvent être pleines, creuses ou poreuses, enrobées ou non.

Parmi les charges utilisables dans les compositions selon l'invention, on peut notamment citer des charges minérales telles que le talc ; le mica naturel ou synthétique ; le kaolin ; le nitrure de bore, le carbonate de calcium précipité ; le carbonate et l'hydro-carbonate de magnésium ; l'hydroxyapatite, l'oxyde de cérium, l'oxyde de zirconium.

Avantageusement, la ou les particules inorganiques présentent une taille primaire moyenne en nombre comprise entre 0,1 et 30 µm, de préférence comprise entre 0,2 et 20 µm, et encore plus préférentiellement comprise entre 0,5 et 15 µm. Au sens de la présente invention, on entend par « taille primaire de particule », la dimension maximale qu'il est possible de mesurer entre deux points diamétralement opposés d'une particule individuelle. La taille des particules organiques peut être déterminée par microscopie électronique à transmission ou à partir de la mesure de la surface spécifique par la méthode BET ou à partir d'une granulométrie laser.

De préférence les charges inorganiques utilisées selon l'invention sont le talc, le nitrure de bore, le dioxyde de titane.

Parmi les charges utilisables dans les compositions selon l'invention, on peut notamment citer des charges organiques. Par charge organique, on entend une particule polymérique qui peut être issue de la polymérisation d'un ou de plusieurs monomères. Les polymères constituant ces particules organiques peuvent être réticulés ou non. Les monomères utilisés peuvent être en particulier des esters d'acide méthacrylique ou acrylique, tels que l'acrylate et méthacrylate de méthyle, le chlorure de vinylidène, l'acrylonitrile, le styrène et ses dérivés.

Avantageusement, la ou les particules organiques présentent une taille primaire moyenne en nombre comprise entre 1 et 30 µm, de préférence comprise entre 1 et 20 µm, et encore plus préférentiellement comprise entre 1 et 15 µm.

La ou les particules organiques utilisées dans la composition cosmétique selon l'invention peuvent être choisies parmi les poudres de polyamide, les poudres de polymères acryliques, notamment de polyméthacrylate de méthyle, les poudres de copolymères acryliques, notamment de polyméthacrylate de méthyle/diméthacrylate d'éthylène glycol, de polyméthacrylate d'allyle / diméthacrylate d'éthylène glycol, de copolymère diméthacrylate d'éthylène glycol / méthacrylate de lauryle, de polyacrylate / alkylacrylate, les poudres de polystyrène, les poudres de polyéthylène, notamment de polyéthylène / acide acrylique.

A titre représentatif et non limitatif, on peut particulièrement citer en tant que particules organiques selon l'invention :
- les poudres de polyamides (Nylon ®), par exemple celles commercialisées sous les dénominations « ORGASOL ® 4000 » et « ORGASOL ® 2002 UD NAT COS 204 » par la société ATOCHEM ;
- les poudres de polymères acryliques, notamment de polyméthacrylate de méthyle, comme par exemple celles commercialisées sous la dénomination « COVABEAD ® LH85 » « COVABEAD ® PMMA »par la société LCW ou celles commercialisées sous la dénomination « MICROPEARL ® MHB » commercialisée par la société MATSUMOTO ;
- les poudres de copolymères acryliques, notamment de polyméthacrylate de méthyle/diméthacrylate d'éthylène glycol, comme celles commercialisées sous la dénomination de « DOW CORNING 5640 MICROSPONGE ® SKIN OIL ADSORBER » par la société DOW CORNING, ou celles commercialisés sous la dénomination « GANZPEARL ® GMP-0820 » par la société GANZ CHEMICAL, de polyméthacrylate d'allyle/diméthacrylate d'éthylène glycol, comme celles commercialisées sous la dénomination « POLYPORE ® L200 » ou « POLYPORE ® E200 » commercialisés par la société AMCOL, de copolymère diméthacrylate d'éthylène glycol / méthacrylate de lauryle, comme celles commercialisées par « POLYTRAP ® 6603 » par la société DOW CORNING, de polyacrylate/éthylhexylacrylate comme celles commercialisées sous la dénomination « TECHPOLYMER ® ACX 806C » par la société SEKISUI ;
- les poudres de polystyrène / dinvinylbenzène comme celles commercialisées sous la dénomination « TECHPOLYMER ® SBX8 » par la société SEKISUI ;
- les poudres de polyéthylène, notamment de polyéthylène/acide acrylique commercialisées sous la dénomination « FLOBEADS ® » par la société SUMITOMO ;
- les microsphères de polymères acryliques telles que celles en copolymère d'acrylate réticulé'POLYTRAP 6603 ADSORBER®' de la société RP SCHERRER ;
- les poudres de polyuréthane telle que la poudre de copolymère de diisocyanate d'hexaméthylène et de triméthylol hexyl lactone vendue sous la dénomination « PLASTIC POWDER D-400® » par la société Toshiki ;
- les microcapsules de polymères ou de copolymères d'acrylate ou de méthacrylate de méthyle, ou encore de copolymères de chlorure de vinylidène et d'acrylonitrile, comme l' « EXPANCEL® » de la société EXPANCEL ;
- les poudres d'organopolysiloxane réticulés élastomères telles que celles vendues sous la dénomination « TREFIL POWDER E-506C » par la société DOW CORNING ;
- les poudres polyfluorées notamment de polytétrafluoroéthylène, par exemple celle commercialisée sous la dénomination "MP 1400" par la Société DUPONT DE NEMOURS.

De préférence, les particules organiques utilisées dans la composition conforme à l'invention sont choisies parmi les poudres de polyamide et les poudres de polyméthylméthacrylate.

Les compositions selon l'invention peuvent aussi comprendre un ou plusieurs agents épaississants des huiles choisis parmi les agents épaississants polymériques, les agents épaississants minéraux et leurs mélanges.

L'agent épaississant polymérique est par exemple un polymère amorphe formé par polymérisation d'une oléfine. L'oléfine peut être notamment un monomère à insaturation éthylénique élastomérique.

Comme exemple d'oléfine, on peut citer les monomères de carbure éthylénique, ayant notamment une ou deux insaturations éthylénique, ayant de 2 à 5 atomes de carbone tels que l'éthylène, le propylène, le butadiène, l'isoprène.

L'agent épaississant polymérique est capable d'épaissir ou de gélifier la phase organique de la composition. Par polymère amorphe, on entend un polymère qui n'a pas de forme cristalline. L'agent épaississant polymérique peut être également filmogène.

L'agent épaississant polymérique peut être notamment un copolymère dibloc, tribloc, multibloc, radial, étoile, ou leurs mélanges.

De tels agents épaississants polymériques sont décrits dans la demande US-A-2002/005562 et dans le brevet US-A-5 221 534

Avantageusement, l'agent épaississant polymérique est un copolymère bloc amorphe de styrène et d'oléfine.

L'agent épaississant polymérique est de préférence hydrogéné pour réduire les insaturations éthyléniques résiduelles après la polymérisation des monomères.

En particulier, l'agent épaississant polymérique est un copolymère, éventuellement hydrogéné, à blocs styrène et à blocs éthylène / alkylène en C₃-C₄.

Comme copolymère dibloc, de préférence hydrogéné, on peut citer les copolymères de styrène-éthylène / propylène, les copolymères de styrène-éthylène / butadiène. Des polymères diblocs sont notamment vendus sous la dénomination Kraton® G1701 E par la société Kraton Polymers.

Comme copolymère tribloc, de préférence hydrogéné, on peut citer les copolymères de styrène-éthylène / propylène-styrène, les copolymères de styrène-éthylène / butadiène-styrène, les copolymères de styrène-isoprène-styrène, les copolymères de styrène-butadiène-styrène. Des polymères triblocs sont notamment vendus sous les dénominations Kraton® G1650, Kraton® G1652, Kraton® D1101, Kraton® D1102, Kraton® D1160 par la société Kraton Polymers.

On peut également utiliser un mélange de copolymère hydrogéné tribloc styrène-butylène / éthylène-styrène et de polymère étoile hydrogéné éthylène-propylène-styrène, un tel mélange étant notamment dans l'isododécane. De tels mélanges sont par exemple vendus par la société PENRECO sous les dénominations commerciales VERSAGEL® M5960 et VERSAGEL® M5670.

Avantageusement, on utilise comme agent épaississant polymérique un copolymère dibloc tel que ceux décrits précédemment, en particulier un copolymère dibloc de styrène-éthylène / propylène.

L'agent épaississant polymérique peut être présent en une teneur allant de 0,1 % à 10 % en poids, par rapport au poids total de la composition, de préférence allant de 0,5 % à 8 % en poids, et plus préférentiellement allant de 1 % à 5 % en poids.

La composition peut également comprendre un ou plusieurs agents épaississants minéraux des huiles tels qu'une argile organophile, les silices pyrogénées.

Les argiles organophiles sont des argiles modifiées par des composés chimiques rendant l'argile apte à gonfler dans les milieux huileux.

Les argiles sont des produits déjà bien connus en soi, qui sont décrits par exemple dans l'ouvrage "Minéralogie des argiles, S. Caillère, S. Hénin, M. Rautureau, 2ème édition 1982, Masson", dont l'enseignement est ici inclus à titre de référence.

Les argiles sont des silicates contenant un cation pouvant être choisi parmi les cations de calcium, de magnésium, d'aluminium, de sodium, de potassium, de lithium et leurs mélanges.

A titre d'exemples de tels produits, on peut citer les argiles de la famille des smectites telles que les montmorillonites, les hectorites, les bentonites, les beidellites, les saponites, ainsi que de la famille des vermiculites, de la stévensite, des chlorites.

Ces argiles peuvent être d'origine naturelle ou synthétique. De préférence, on utilise les argiles qui sont cosmétiquement compatibles et acceptables avec les matières kératiniques.

L'argile organophile peut être choisie parmi la montmorrilonite, la bentonite, l'hectorite, l'attapulgite, la sépiolite, et leurs mélanges. L'argile est de préférence une bentonite ou une hectorite.

Ces argiles peuvent être modifiées avec un composé chimique choisi parmi les amines quaternaires, les amines tertiaires, les acétates aminés, les imidazolines, les savons aminés, les sulfates gras, les alkyl aryl sulfonates, les oxides amines, et leurs mélanges.

Comme argiles organophiles, on peut citer les quaternium-18 bentonites telles que celles vendues sous les dénominations Bentone 3, Bentone 38, Bentone 38V par la société Rhéox, Tixogel VP par la société United catalyst, Claytone 34, Claytone 40, Claytone XL par la société Southern Clay; les stéaralkonium bentonites telles que celles vendues sous les dénominations Bentone 27 par la société Rheox, Tixogel LG par la société United Catalyst, Claytone AF, Claytone APA par la société Southern Clay ; les quaternium-18/benzalkonium bentonite telles que celles vendues sous les dénominations Claytone HT, Claytone PS par la société Southern Clay.

Les silices pyrogénées peuvent être obtenues par hydrolyse à haute température d'un composé volatil du silicium dans une flamme oxhydrique, produisant une silice finement divisée. Ce procédé permet notamment d'obtenir des silices hydrophiles qui présentent un nombre important de groupements silanol à leur surface. De telles silices hydrophiles sont par exemple commercialisées sous les dénominations "AEROSIL 130®", "AEROSIL 200®", "AEROSIL 255®", "AEROSIL 300®", "AEROSIL 380®" par la société Degussa, "CAB-O-SIL HS-5®", "CAB-O-SIL EH-5®", "CAB-O-SIL LM-130®", "CAB-O-SIL MS-55®", "CAB-O-SIL M-5®" par la société Cabot.

Il est possible de modifier chimiquement la surface de ladite silice, par réaction chimique générant une diminution du nombre de groupes silanol. On peut notamment substituer des groupes silanol par des groupements hydrophobes : on obtient alors une silice hydrophobe.

Les groupements hydrophobes peuvent être :
- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6^{ème} édition, 1995). Elles sont par exemple commercialisées sous les références "AEROSIL R812®" par la société Degussa, "CAB-O-SIL TS-530®" par la société Cabot ;
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénomées "Silica diméthyl silylate" selon le CTFA (6^{ème} édition, 1995). Elles sont par exemple commercialisées sous les références "AEROSIL R972®", "AEROSIL R974®" par la société Degussa, "CAB-O-SIL TS-610®", "CAB-O-SIL TS-720®" par la société Cabot.

La silice pyrogénée présente de préférence une taille de particules pouvant être nanométrique à micrométrique, par exemple allant d'environ de 5 à 200 nm.

De préférence, on utilise comme agent épaississant minéral une hectorite ou une bentonite organomodifiée.

L'agent épaississant minéral des huiles peut être présent dans la composition en une teneur allant de 0,1 % à 8 % en poids, par rapport au poids total de la composition, de préférence en une teneur allant de 0,2 % à 6 % en poids, et préférentiellement allant de 0,5 % à 4 % en poids.

Les compositions conformes à l'invention peuvent également contenir au moins un agent utilisé habituellement en cosmétique, choisi, par exemple, parmi des agents réducteurs, des corps gras, des plastifiants, des adoucissants, des agents anti-mousse, des agents hydratants, des filtres UV, des colloïdes minéraux, des peptisants, des solubilisants, des parfums, des protéines, des vitamines, des propulseurs, les cires oxyéthylénées ou non, les paraffines, les acides gras en C₁₀-C₃₀ tels que l'acide stéarique, l'acide laurique, les amides gras en C₁₀-C₃₀ tel que le diéthanolamide laurique.

Les additifs ci dessus sont en général présents en quantité comprise pour chacun d'eux entre 0,01 et 20 % en poids par rapport au poids de la composition.

Bien entendu, l'homme de l'art veillera à choisir ce ou ces éventuels additifs de manière telle que les propriétés avantageuses attachées intrinsèquement à la formation du gainage conforme à l'invention ne soient pas, ou substantiellement pas, altérées.

La composition selon l'invention peut se présenter notamment sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de mousse, de stick, de dispersion de vésicules notamment de lipides ioniques ou non, de lotion biphase ou multiphase, de spray, de poudre, de pâte.

La composition peut être une composition anhydre, c'est-à-dire une composition contenant moins de 2 % en poids d'eau, voire moins de 0,5 % d'eau, notamment exempte d'eau, l'eau n'étant pas ajoutée lors de la préparation de la composition mais correspondant à l'eau résiduelle apportée par les ingrédients mélangés. La composition peut également se présenter sous forme de laque.

De préférence, la composition conforme à l'invention est anhydre.

La composition décrite ci-dessus peut être mise en oeuvre sur cheveux secs ou humides. Les additifs décrits précédemment, lorsqu'ils sont présents, peuvent être appliqués sur les cheveux simultanément avec la composition de l'invention ou séparément. La composition peut être rincée ou non. Il est aussi possible d'effectuer ensuite un lavage des cheveux, ce lavage n'étant pas obligatoire.

On peut également utiliser un procédé d'application avec chauffage. Selon ce mode particulier, l'application sur les cheveux est mise en oeuvre par exemple au moyen d'un peigne, d'un pinceau à l'aide d'une brosse ou aux doigts.

L'application de la composition est ensuite suivie d'un séchage à une température supérieure à 40 °C. Selon un mode de réalisation particulier, cette température est supérieure à 45 °C. Selon un autre mode de réalisation particulier, cette température est supérieure à 45 °C et inférieure à 220 °C.

Le séchage peut être réalisé immédiatement après l'application ou après un temps de pose pouvant aller de 1 minute à 30 minutes.

De préférence, les cheveux sont séchés, en plus d'un apport de chaleur, avec un flux d'air. Ce flux d'air pendant le séchage permet d'améliorer l'individualisation du gainage.

Durant le séchage, une action mécanique sur les mèches peut être exercée telle qu'un peignage, un brossage, le passage des doigts.

L'étape de séchage du procédé de l'invention peut être mise en oeuvre avec un casque, un sèche-cheveux, un fer à lisser, un climazon, ...

Lorsque l'étape de séchage est mise en oeuvre avec un casque ou un sèche-cheveux, la température du séchage est comprise entre 40 et 110 degrés, de préférence entre 50 et 90 degrés.

Lorsque l'étape de séchage est mise en oeuvre avec un fer à lisser, la température du séchage est comprise entre 110 et 220 degrés, de préférence entre 140 et 200 degrés.

Une fois le séchage terminé, un rinçage ou un shampooing terminal peut éventuellement être réalisé.

La présente invention a également pour objet l'utilisation d'une composition telle que définie précédemment comprenant un ou plusieurs pigments colorés pour l'obtention de gainage coloré sur les cheveux.

La présente invention a aussi pour objet l'utilisation d'une composition telle que définie précédemment ne comprenant pas de pigments pour le traitement des cheveux.

L'invention va être plus complètement illustrée à l'aide des exemples non limitatifs suivants.

### EXEMPLES

### Exemple 1 :

La composition suivante est réalisée :

| Composition | A |
|---|---|
| Silicone cyclique volatile DC245 Fluid (*) | 81 g |
| Polyméthylsilsesquioxane commercialisé sous le nom Wacker Belsil PMS MK Powder par la société Wacker | 2 g |
| Mélange Polydimethylsiloxane alpha-omega dihydroxyle / Cyclopentadimethylsiloxane (14,7 / 85,3) commercialisé sous le nom DC1501 Fluid (*) | 7 g |
| BioPSA 7-4405 (BioPSA 7-4400 dilué à 40 % dans l'isododécane) (*) | 15 g |

| | |
|---|---|
| (*) commercialisé par Dow Corning | |

0,3 g de la composition est appliqué sur une mèche de 1 g de cheveux de hauteur de ton 4 propres et humides. Après 15 minutes de pause, la mèche est séchée au sèche-cheveux pendant 2 minutes. On obtient une mèche dont les cheveux sont individualisés et gainés. Ce gainage est rémanent au shampooing.

### Exemple 2 :

La composition suivante est réalisée :

| Composition | B |
|---|---|
| Isododécane | 79 g |
| Polypropylsilsesquioxane dilué à 50 % en poids dans D5 commercialisé sous le nom DC670 Fluid (*) | 4 g |
| Silicone linéaire DC200 Fluid 500.000 cst (*) | 2 g |
| BioPSA 7-4405 (BioPSA 7-4400 dilué à 40% dans l'isododécane) (*) | 15 g |

| | |
|---|---|
| (*) commercialisé par Dow Corning | |

0,3 g de la composition est appliqué sur une mèche de 1 g de cheveux de hauteur de ton 4 propres et humides. Après 15 minutes de pause, la mèche est séchée au sèche-cheveux pendant 2 minutes. On obtient une mèche dont les cheveux sont individualisés et gainés. Ce gainage est rémanent au shampooing.

### Exemple 3 :

La composition suivante est réalisée :

| Composition | C1 | C2 |
|---|---|---|
| Silicone cyclique volatile DC245 Fluid (*) | 42 g | 45 g |
| Mélange Polydimethylsiloxane alpha-omega dihydroxyle / Cyclopentadimethylsiloxane (14,7 / 85,3) commercialisé sous le nom DC1501 Fluid (*) | 10 g | 10 g |
| Polyméthylsilsesquioxane commercialisé sous le nom Wacker Belsil PMS MK Powder par la société Wacker | 3g | - |
| Nacre mica enrobé d'oxyde de fer brun commercialisée par Eckart sous le nom Prestige Bronze | 10 g | 10 g |
| Disteardimonium hectorite (10%) et propylene carbonate (3%) dans l'isododécane commercialisé par Elementis sous le nom Bentone Gel ISD V | 15 g | 15 g |
| BioPSA 7-4405 (BioPSA 7-4400 dilué à 40% dans l'isododécane) (*) | 20 g | 20 g |

| | | |
|---|---|---|
| (*) commercialisé par Dow Corning | | |

Pour les compositions C1 et C2, 0,5 g de la composition est appliqué sur une mèche de 1 g de cheveux de hauteur de ton 4 propres et humides. Après 15 minutes de pause, la mèche est séchée au sèche-cheveux pendant 2 minutes. On obtient avec chacune des compostions une mèche colorée dont les cheveux sont individualisés et dont la couleur est rémanente au shampooing. Cependant, le toucher de la mèche obtenue avec la composition C1 est moins collant que le toucher de la mèche obtenue avec la composition C2.

### Exemple 4 :

La composition suivante est réalisée :

| Composition | D |
|---|---|
| Silicone cyclique volatile DC245 Fluid (*) | 39 g |
| Mélange Polydimethylsiloxane alpha-omega dihydroxyle / Cyclopentadimethylsiloxane (14,7 / 85,3) commercialisé sous le nom DC1501 Fluid (*) | 10 g |
| Polypropylsilsesquioxane dilué à 50 % en poids dans D5 commercialisé sous le nom DC670 Fluid (*) | 6 g |
| Nacre mica enrobé d'oxyde de fer brun commercialisée par Eckart sous le nom Prestige Bronze | 10 g |
| Disteardimonium hectorite (10%) et propylene carbonate (3%) dans l'isododécane commercialisé par Elementis sous le nom Bentone Gel ISD V | 15 g |
| BioPSA 7-4405 (BioPSA 7-4400 dilué à 40% dans l'isododécane) (*) | 20 g |

| | |
|---|---|
| (*) commercialisé par Dow Corning | |

0,5 g de la composition est appliqué sur une mèche de 1 g de cheveux de hauteur de ton 4 propres et humides. Après 15 minutes de pause, la mèche est séchée au sèche-cheveux pendant 2 minutes. On obtient une mèche colorée dont les cheveux sont individualisés et dont la couleur est rémanente au shampooing.

### Exemple 5 :

La composition suivante est réalisée :

| Composition | E |
|---|---|
| Isododécane | 50 g |
| Silicone linéaire DC200 Fluid 500.000 cst (*) | 2 g |
| Polyméthylsilsesquioxane commercialisé sous le nom Wacker Belsil PMS MK Powder par la société Wacker | 3 g |
| Nacre mica enrobé d'oxyde de fer brun commercialisée par Eckart sous le nom Prestige Bronze | 10 g |
| Disteardimonium hectorite (10 %) et propylene carbonate (3 %) dans l'isododécane commercialisé par Elementis sous le nom Bentone Gel ISD V | 15g |
| BioPSA 7-4405 (BioPSA 7-4400 dilué à 40% dans l'isododécane) (*) | 20 g |

| | |
|---|---|
| (*) commercialisé par Dow Corning | |

0,5 g de la composition est appliqué sur une mèche de 1 g de cheveux de hauteur de ton 4 propres et humides. Après 15 minutes de pause, la mèche est séchée au sèche-cheveux pendant 2 minutes. On obtient une mèche colorée dont les cheveux sont individualisés et dont la couleur est rémanente au shampooing.

## Revendications

1. Composition de traitement des fibres kératiniques comprenant un ou plusieurs copolymères à base de résine de silicone et de silicone fluide, un ou plusieurs solvants volatils, et une ou plusieurs résines de silicone ayant au moins une unité trifonctionnelle de formule (CH₃)SiO_{3/2}.

2. Composition selon la revendication 1 dans laquelle le copolymère comprend la résine de silicone à un taux massique compris entre 45 et 75 % par rapport à la masse totale de silicone et la silicone fluide à un taux massique compris entre 25 et 55 % par rapport à la masse totale de silicone, avec la somme des pourcentages de résine de silicone et de silicone fluide qui est égal à 100.

3. Composition selon la revendication 1 ou 2 dans laquelle le ou les solvants volatils sont choisis parmi les solvants organiques non siliconés et les solvants organiques siliconés.

4. Composition selon l'une quelconque des revendications précédentes dans laquelle le ou les solvants volatils sont choisis parmi l'éthanol, l'isopropanol, l'acétone, les alcanes liquides à 25 °C et à pression atmosphérique (760 mmHg), la décaméthylcyclopentasiloxane, l'octaméthyltrisiloxane, la décaméthyltétrasiloxane et la dodécaméthylcyclohexasiloxane ou leurs mélanges.

5. Composition selon la revendication 4 dans laquelle l'alcane liquide à 25 °C et à pression atmosphérique (760 mmHg) est l'isododécane.

6. Composition selon l'une quelconque des revendications précédentes dans laquelle la ou les résines de silicone sont choisies parmi les silsesquioxanes qui sont représentées par la formule suivante : ((CH₃)SiO_{3/2})ₓ, où x est compris entre 100 et 500 et le radical CH₃ peut être remplacé par un radical R représentant un radical hydrocarboné ayant de 2 à 10 atomes de carbone différent du radical méthyle ou un radical phényle ou un radical hydroxyle.

7. Composition selon l'une quelconque des revendications précédentes dans laquelle le poids moléculaire de la ou des résines de silicone est compris entre 500 et 50 000 g/mol.

8. Composition selon l'une quelconque des revendications précédentes dans laquelle la ou les résines de silicone sont choisies parmi les polyméthylsilsesquioxanes qui sont des polysilsesquioxanes dans lesquels aucun des radicaux méthyle n'est substitué par un autre groupement ; les polypropylsilsesquioxanes pour lesquelles les radicaux méthyle sont remplacés par des radicaux propyle ; les polyphénylsilsesquioxanes pour lesquelles les radicaux méthyle sont remplacés par des radicaux phényle.

9. Composition selon l'une quelconque des revendications précédentes comprenant un ou plusieurs pigments.

10. Composition selon la revendication 9 dans laquelle le ou les pigments sont des pigments minéraux.

11. Composition selon la revendication 10 dans laquelle le ou les pigments sont choisis parmi les oxydes de fer ou de chrome, le violet de manganèse, le bleu outremer, l'hydrate de chrome et le bleu ferrique, le dioxyde de titane.

12. Composition selon la revendication 9 dans laquelle le ou les pigments sont des nacres.

13. Composition selon l'une quelconque des revendications précédentes anhydre.

14. Procédé de traitement des fibres kératiniques comprenant l'application d'une composition telle que définie à l'une quelconque des revendications 1 à 13, éventuellement suivi après un temps de pose d'un rinçage et / ou lavage et / ou séchage à une température supérieure à 40 °C.

15. Utilisation d'une composition telle que définie à l'une quelconque des revendications 1 à 13 pour le traitement des cheveux.

## Patentansprüche

1. Zusammensetzung zur Behandlung von Keratinfasern, umfassend ein oder mehrere Copolymere auf Basis von Silikonharz und fließfähigem Silikon, ein oder mehrere flüchtige Lösungsmittel und ein oder mehrere Silikonharze mit mindestens einer trifunktionellen Einheit der Formel (CH₃)SiO_{3/2}.

2. Zusammensetzung nach Anspruch 1, wobei das Copolymer das Silikonharz in einem Gewichtsanteil zwischen 45 und 75%, bezogen auf das Gesamtgewicht an Silikon, und das fließfähige Silikon in einem Gewichtsanteil zwischen 25 und 55%, bezogen auf das Gesamtgewicht an Silikon, umfasst, wobei die Summe der Prozentanteile des Silikonharzes und des fließfähigen Silikons gleich 100 ist.

3. Zusammensetzung nach Anspruch 1 oder 2, wobei das bzw. die flüchtigen Lösungsmittel aus organischen Nichtsilikon-Lösungsmitteln und organischen Silikon-Lösungsmitteln ausgewählt sind.

4. Zusammensetzung nach einem der vorhergehenden Ansprüche, wobei das bzw. die flüchtigen Lösungsmittel aus Ethanol, Isopropanol, Aceton, Alkanen, die bei 25°C und Normaldruck (760 mmHg) flüssig sind, Decamethylcyclopentasiloxan, Octamethyltrisiloxan, Decamethyltetrasiloxan und Dodecamethylcyclohexasiloxan oder Mischungen davon ausgewählt sind.

5. Zusammensetzung nach Anspruch 4, wobei es sich bei dem Alkan, das bei 25°C und Normaldruck (760 mmHg) flüssig ist, um Isododecan handelt.

6. Zusammensetzung nach einem der vorhergehenden Ansprüche, wobei das bzw. die Silikonharze aus Silsesquioxanen der folgenden Formel ausgewählt sind: ((CH₃)SiO_{3/2})ₓ, wobei x zwischen 100 und 500 liegt und der CH₃-Rest durch einen R-Rest, der für einen Kohlenwasserstoffrest mit 2 bis 10 Kohlenstoffatomen, der von einem Methylrest verschieden ist, oder einen Phenylrest oder einen Hydroxylrest steht, ersetzt sein kann.

7. Zusammensetzung nach einem der vorhergehenden Ansprüche, wobei das Molekulargewicht des bzw. der Silikonharze zwischen 500 und 50.000 g/mol liegt.

8. Zusammensetzung nach einem der vorhergehenden Ansprüche, wobei das bzw. die Silikonharze aus Polymethylsilsesquioxanen, in denen keiner der Methylreste durch eine andere Gruppe substituiert ist; Polypropylsilsesquioxanen, für die die Methylreste durch Propylreste ersetzt sind; Polyphenylsilsesquioxanen, für die die Methylreste durch Phenylreste ersetzt sind; ausgewählt sind.

9. Zusammensetzung nach einem der vorhergehenden Ansprüche, die ein oder mehrere Pigmente umfasst.

10. Zusammensetzung nach Anspruch 9, wobei es sich bei dem bzw. den Pigmenten um anorganische Pigmente handelt.

11. Zusammensetzung nach Anspruch 10, bei dem das bzw. die Pigmente aus Eisen- oder Chromoxiden, Manganviolett, Ultramarinblau, Chromhydrat und Eisen(III)-Blau, Titandioxid ausgewählt sind.

12. Zusammensetzung nach Anspruch 9, wobei es sich bei dem bzw. den Pigmenten um Perlmutt handelt.

13. Zusammensetzung nach einem der vorhergehenden Ansprüche, die wasserfrei ist.

14. Verfahren zur Behandlung von Keratinfasern, bei dem man eine Zusammensetzung gemäß einem der Ansprüche 1 bis 13 aufbringt und dann gegebenenfalls nach einer Einwirkungszeit spült und/oder wäscht und/oder bei einer Temperatur von mehr als 40°C trocknet.

15. Verwendung einer Zusammensetzung gemäß einem der Ansprüche 1 bis 13 zur Behandlung von Haaren.

## Claims

1. Composition for treating keratin fibres, comprising one or more copolymers based on silicone resin and fluid silicone, one or more volatile solvents, and one or more silicone resins containing at least one trifunctional unit of formula (CH₃)SiO_{3/2}.

2. Composition according to Claim 1, in which the copolymer comprises the silicone resin in a mass content of between 45% and 75% relative to the total mass of silicone and the fluid silicone in a mass content of between 25% and 55% relative to the total mass of silicone, the sum of the percentages of silicone resin and of fluid silicone being equal to 100.

3. Composition according to Claim 1 or 2, in which the volatile solvent(s) is (are) chosen from non-silicone organic solvents and silicone organic solvents.

4. Composition according to any one of the preceding claims, in which the volatile solvent(s) is (are) chosen from ethanol, isopropanol, acetone, alkanes that are liquid at 25°C and at atmospheric pressure (760 mmHg), decamethylcyclopentasiloxane, octamethyltrisiloxane, decamethyltetrasiloxane and dodecamethyl-cyclohexasiloxane, or mixtures thereof.

5. Composition according to Claim 4, in which the alkane that is liquid at 25°C and at atmospheric pressure (760 mmHg) is isododecane.

6. Composition according to any one of the preceding claims, in which the silicone resin(s) is (are) chosen from the silsesquioxanes that are represented by the following formula: ((CH₃)SiO_{3/2})ₓ, in which x is between 100 and 500 and the radical CH₃ may be replaced with a radical R representing a hydrocarbon-based radical containing from 2 to 10 carbon atoms, other than a methyl radical, a phenyl radical or a hydroxyl radical.

7. Composition according to any one of the preceding claims, in which the molecular weight of the silicone resin(s) is between 500 and 50 000 g/mol.

8. Composition according to any one of the preceding claims, in which the silicone resin(s) is (are) chosen from polymethylsilsesquioxanes, which are polysilsesquioxanes in which none of the methyl radicals is substituted with another group; polypropylsilsesquioxanes, for which the methyl radicals are replaced with propyl radicals; polyphenylsilsesquioxanes, for which the methyl radicals are replaced with phenyl radicals.

9. Composition according to any one of the preceding claims comprising one or more pigments.

10. Composition according to Claim 9, in which the pigment(s) are natural pigments.

11. Composition according to Claim 10, in which the pigment or pigments are chosen from iron oxide, chromium oxide, manganese violet, ultramarine blue, chromium hydrate and ferric blue, titanium dioxide.

12. Composition according to Claim 9, in which the pigment or pigments are nacres.

13. Composition according to any one of the preceding claims, which is anhydrous.

14. Process for treating keratin fibres comprising the application of a composition as defined in any one of Claims 1 to 13, optionally followed, after a leave-in time, by rinsing and/or washing and/or drying at a temperature above 40°C.

15. Use of a composition as defined in any of Claims 1 to 13 for treating the hair.
